# EUROPEAN PATENT APPLICATION

(11) **EP 2 372 365 A1**
(43) Date of publication of application: **05.10.2011**
(21) Application number: 10159035.4
(22) Date of filing: 01.04.2010
(51) Int. Cl.: G01N 33/82

(54) **Direct immunoassay for vitamin D**

(71) Applicant: Future Diagnostics B.V., 6603 BT Wijchen (NL)
(72) Inventor: Martens, Michaël Franciscus Wilhelmus Cornelis, 5704 HC Helmond (NL); Parsons, George Henry, Arlington, MA 02476 (US); Rosmalen, Franciscus Maria Anna, 6602 EP Wychen (NL); Swinkels, Leon Maria Jacobus Wilhelmus, 6881 DA Bemmel (NL)
(74) Representative: Hatzmann, Martin

(57) **Abstract**

Disclosed is the invention to conduct immuno-adsorption of 25(OH) vitamin D from blood or blood components, notably serum or plasma, after which the absorbed material is measured. The invention thus employs a binding protein, preferably an radiolabels, fluorescent labels, luminescent labels, biotin labels, gold labels, enzyme labels, for 25(OH) vitamin D in affecting displacement of said vitamin D from vitamin D binding protein. Thereafter the binding protein comprising the 25-OH vitamin D is subjected to a competitive binding assay with a labeled vitamin D compound. The method is suitable for both measuring total vitamin D and free vitamin D.

## Description

### Field of the invention

The invention pertains to a direct method for assaying a sample of blood or blood components for the presence of 25-hydroxy vitamin D. The invention also relates to immunoassays and kits for conducting such immunoassays including point-of-care tests.

### Background of the invention

The substances referred to as "vitamin D" encompass a group of fat-soluble prohormones, as well as metabolites and analogues thereof. The main forms in which vitamin D occurs in the body are vitamin D₂ (ergocalciferol) and vitamin D₃ (cholecalciferol). The latter is the endogenous form of vitamin D, which humans can form in the skin under the influence of sunlight. The former is an exogenous form of vitamin D, taken up with food. In the US, Vitamin D2 is used as the pharmaceutical vitamin D supplement.

Whilst vitamin D₂ and D₃ differ in the molecular structure of their side-chains, they share the same biological activity in being prohormones, metabolized in two steps to, ultimately, 1,25 dihydroxy vitamin D (calcitriol, or 1,25 dihydroxy cholecalciferol). The preceding metabolite, 25-hydroxy vitamin D or calcidiol, results from conversion in the liver, and is considered the storage form of vitamin D in the body.

The serum level of 25-hydroxy-vitamin D is considered to be the primary indicator of the vitamin D status. Circulating vitamin D consists mainly of 25(OH)vitamin D3 and 25(OH)vitamin D2. Biologically, 25(OH)vitamin D2 is as effective as 25(OH)vitamin D3. The half-life of 25(OH)vitamin D2 in the circulation is shorter. For clinical practice the use of 25(OH)vitamin D assay that measures both 25(OH)vitamin D3 as well as 25(OH)vitamin D2 is recommended.

Vitamin D has long been recognized as an important substance, the active form of which plays a role in the formation and maintenance of bone, as well as in other processes in the human or animal body. Thus, it serves to increase the flow of calcium into the bloodstream, by promoting absorption of calcium and phosphorus from food in the intestines, and reabsorption of calcium in the kidneys; enabling normal mineralization of bone and preventing hypocalcemic tetany. It is also necessary for bone growth and bone remodeling by osteoblasts and osteoclasts.

Vitamin D deficiency results in impaired bone mineralization and leads to bone softening diseases, rickets in children and osteomalacia in adults, and possibly contributes to osteoporosis.

Vitamin D plays a number of other roles in human health including inhibition of calcitonin release from the thyroid gland. Calcitonin acts directly on osteoclasts, resulting in inhibition of bone resorption and cartilage degradation. Vitamin D can also inhibit parathyroid hormone secretion from the parathyroid gland, modulate neuromuscular and immune function and reduce inflammation. Thus, it is of the essence for a person's or animal's health to have an adequate level of vitamin D.

Yet, excess of vitamin D (which may occur as a result of overdosing) is toxic. Some symptoms of vitamin D toxicity are hypercalcaemia (an elevated level of calcium in the blood) caused by increased intestinal calcium absorption. Vitamin D toxicity is known to be a cause of high blood pressure. Gastrointestinal symptoms of vitamin D toxicity can include anorexia, nausea, and vomiting. These symptoms are often followed by polyuria (excessive production of urine), polydipsia

(increased thirst), weakness, nervousness, pruritus (itch), and eventually renal failure.

Clearly, it is important to be able to diagnose subjects for a possible vitamin D deficiency. It is also important, particularly for subjects that are on vitamin D supplementation, to be able to test subjects for a potential excess of vitamin D.

To this end a variety of assays for the quantitative determination of vitamin D exists. A major consideration for such assays stems from the fact that the almost all circulating 25(OH)-vitamin D in serum is bound by vitamin D binding protein (88%) and Albumin (12%).

Vitamin D binding protein (DBP) is a major component of serum, with a concentration of 250-400 mg/L of serum. Only a small portion, about 2%, of the binding sites of DBP is occupied. A very small fraction, 0.04%, of the 25(OH) vitamin D circulates in the free, non-protein bound form. DBP represents an interfering factor in quantitative immunoassays or protein binding assays that are based on competition for a limited number of binding sites. To measure Vitamin D accurately, it must be released from the binding protein.

Methods for the determination of circulating 25(OH)-vitamin D therefore rely on the elimination of the binding of 25(OH)-vitamin D to endogenous binding proteins. Binding proteins can be removed by precipitation using water miscible solvent such as ethanol or acetonitrile. After centrifugation, the supernatant can be used for the assay of 25(OH)-vitamin D. 25 (OH)-vitamin D can be extracted using organic solvents such as diethyl ether. After evaporation of the solvent the residue can be dissolved in a buffer for further assay. Extraction can also be achieved using reversed phase chromatography cartridges. Alternatively competitive displacers can be used that bind to the binding protein but not to the antibody.

The existing assays are relatively cumbersome, and particularly unsuitable for so-called "point-of-care" testing. The latter refer to testing at or near the site of patient care, i.e. rather than drawing blood samples and sending these to a diagnostic laboratory, a sample can be immediately introduced into a portable, preferably handheld device which is able to perform the assay in as limited a number of steps as possible, and with as limited a number of manual operations as possible. E.g. an assay in which a sample is first to be subjected to a chemical treatment, such as applying reagents to release vitamin D from DBP, will be too complicated, and prone to error.

In fact, the existing different approaches for the determination of vitamin D in an immunoassay, have in common that they are laborious, and involve techniques such as centrifugation, that are incompatible with assays intended for automated platforms.

It is therefore desired to provide an immunoassay for the direct determination of vitamin D, i.e. without the requirement of a pre- treatment step for releasing the vitamin D from DBP.

Many methods have been developed for assaying circulating 25-hydroxy vitamin D. Some of these assays are competitive protein-binding assays, involving the vitamin D-binding protein from various animal species (6-10), and most require a pretreatment of the sample before assay. See, e.g., Hollis et al., Clin.Chem 39/3, 529-533 (1993).

WO 2008/092917 concerns a method for quantitating vitamin D metabolites directly in blood plasma or serum, stating that this is without the need for prior purification of the vitamin D metabolites. Whilst this method is referred to as a "direct" method, it still involves a separate step of releasing vitamin D from DBP, the essence of which is to add an effective amount of a serine protease with endo- and exoproteolytic activity to a sample containing blood plasma or serum and carrying out a digestion of the vitamin D binding proteins in the blood plasma or serum until they can no longer bind any vitamin D metabolites.

Further, an assay of vitamin D without the need of extraction has been described in US 5,981,779. This concerns the assay of vitamin D in milk samples. As is acknowledged in US 7,482,162, the method described is not suitable for measurement of Vitamin D in serum.

### Summary of the invention

In order to better address one or more of the foregoing desires the invention, in one aspect, presents a method for assaying a sample of blood or blood components for the presence of 25-OH vitamin D, comprising
(a) adding a binding protein for 25-OH vitamin D to the sample;
(b) incubating the sample for an effective amount of time to allow a desired amount of 25-OH vitamin D to bind to the binding protein;
(c) subjecting the binding protein comprising 25-OH vitamin D bound thereto, to competitive binding with a labeled vitamin D compound;
(d) determining the concentration of labeled vitamin D compound bound to the binding protein.

In another aspect, the invention resides in a kit for conducting the foregoing method.

In yet another aspect, the invention presents the use of a binding protein for 25-OH vitamin D for securing the displacement of 25-OH vitamin D from vitamin D Binding Protein in an immunoassay for the determination of 25-OH vitamin D in blood or blood components.

In a still further aspect, the invention puts to use the above assaying method in determining the concentration in the sample of total 25-hydroxy vitamin D. In yet another aspect, the invention puts to use the above assaying method in determining the concentration in the sample of bio-available 25-hydroxy vitamin D.

In another aspect, the invention provides the use of the above method as a pretreatment of a sample of blood or blood components for the determination of bio-available vitamin D by GC-MS.

### Description of drawing

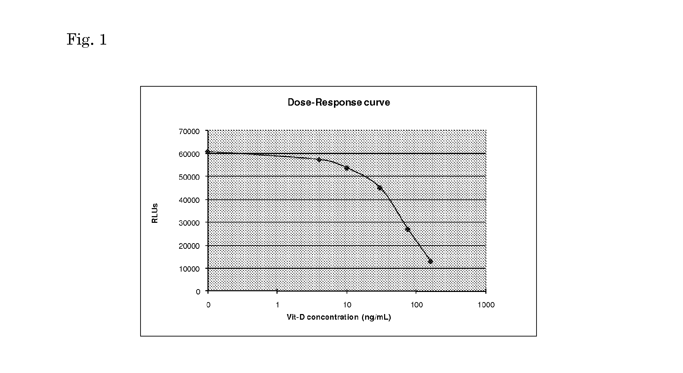

Fig. 1 represents a dose response curve from an assay according to the invention.

### Detailed Description of the Invention

In a broad sense, the invention concerns the immuno-adsorption of 25(OH) vitamin D from blood or blood components, notably serum or plasma, after which the absorbed material is measured. The invention thus employs a binding protein as could be used in a competitive binding assay for the determination of 25-OH vitamin D of the conventional type (in which the vitamin D first needs to be released from DBP), as an *in situ* agent for the release of vitamin D from DBP. This circumvents the addition of a separate releasing agent for vitamin D, and involves a cascade of advantages.

Initially, it is no longer necessary to add any additional chemical such as would be required for the exposure or the release of vitamin D. This constitutes an advantage *per se,* among others in terms of process and costs efficiency. Further, the method in which the binding protein works to bind the vitamin D, in equilibrium with DBP, means that mainly time is required, rather than that a step is to be conducted which, in effect, means a chemical reaction, with the necessary auxiliary steps such as centrifuging. This provides an important benefit in that the assaying method of the invention can be presented in a form suitable for point-of-care diagnostics and/or for automated assay platforms.

Moreover, the use of the binding protein to bind vitamin D also means that the sample automatically already contains the key component for conducting the subsequent competitive binding assay.

The method of the invention is used for assaying the presence of 25-OH vitamin D in a sample of blood or blood components. Particularly, the sample comprises serum or plasma, and preferably serum. Such samples can be drawn, in any manner known in the art, from a subject, particularly a human, in whose blood it is desired to assay the presence of 25-OH vitamin D.

To the sample a binding protein for 25-OH vitamin D is added. Binding proteins, e.g. antibodies, for vitamin D are known in the art, and are widely used in the existing immunoassays for vitamin D. These same antibodies, as well as other binding proteins, can be used in the present invention as well. E.g., in the place of an antibody for Vitamin D an antibody fragment can be used such as produced with phage display technology. Also, in another embodiment, the binding protein used in the assay of the invention can be DBP immobilized on a solid phase.

Suitable antibodies can be monoclonal or polyclonal antibodies. They can be obtained in known manner, e.g. polyclonal goat anti-vitamin D, polyclonal rabbit anti-vitamin D, or any other suitable antibody for vitamin D as known in the art from application in immunoassays for vitamin D. Suitable antibodies are known, e.g. from the following references: Hollis, Clin.Chem 31/11, 1815-1819 (1985); Hollis, Clin.Chem 39/3, 529-533 (1993).

The binding proteins are preferably added in a particulate form comprising solid carriers. Typically, the binding protein is coated on a solid phase, e.g. on a microtiter plate. In a preferred embodiment, the binding protein is coated onto magnetic particles, which facilitates their separation in a magnetic field.

The sample is preferably diluted, before, during, or after the addition of the binding protein. The sample diluent can be aqueous-based, and preferably will be a buffer solution. Preferably, the buffered pH is in the range of from 6.0 to 8.0. Suitable diluents include, e.g. phosphate citrate buffer. Suitable buffer solutions are customary in the art and do not require elucidation here.

After addition of the binding protein, e.g. an antibody, the sample is allowed to incubate. The required time will depend on circumstances such as the concentration of the reagents, the type of binding protein, and conditions during incubation, e.g. shaking and temperature. Generally, the incubation time will be in a range of from 10 seconds to several hours, preferably 1 minute 1 hour. For automated platforms, short incubation times (10 seconds to 10 minutes, preferably 30 seconds to 30 minutes) are preferred. Basically, the period of time is not of particular relevance, as long as one determines in a calibration system how much of the vitamin D is to be bound under the circumstances, during the desired period of time. Or, otherwise, one can decide on the amount of vitamin D that is desired to be bound, and then determine the necessary time to accomplish this. Since the binding of vitamin D to the binding protein will be in equilibrium with the binding of vitamin D to endogenous DBP, any longer period of time will not be particularly effective, as going beyond the time needed for the equilibrium to have been established, will not substantially affect the bound concentrations. Shorter periods of time, i.e. not necessarily leading to a final state of equilibrium, are expressly possible. The main requirement is that of proper calibration. Thus, preferably, comparison with calibrators involves the same period of time, under the same conditions.

After the incubation period, the sample can be subjected in known manner to a competitive binding assay using a labeled vitamin D compound. Numerous labeled compounds are known that are capable of serving as competitive binding antigens in immunoassays for the determination of vitamin D. Typical labels are radiolabels, fluorescent labels, luminescent labels, biotin labels, gold labels, enzyme labels. Competitive binding assays are known to the skilled person, and do not require elucidation, notably since this part of the method of the invention can be carried out using any label known to be suitable for the determination of vitamin D. Labels that can be used are, inter alia, those disclosed in the foregoing references on existing vitamin D immunoassays.

With the label allowing measuring a concentration, as a result, the concentration of vitamin D in the sample is determined. It will be understood that the interpretation of the values measured, is determined by a calibration measurement, i.e. by the response - in the same assay - of calibrators. The calibration for the assay of the invention can be done by providing calibrators comprising a predetermined concentration of 25-OH vitamin D.

The assay of the invention can suitably be employed to determine either total 25-OH vitamin D in a sample, or only bio-available 25-OH vitamin D. This is a matter of calculation, i.e. a value determined upon calibration can be used as a reference for either the total or only the unbound fraction of 25-OH vitamin D.

Measurement of free vitamin D relies on the assessment of the concentration of free Vitamin D without substantially affecting the equilibrium between bound and free Vitamin. Thus, for measuring unbound vitamin D it is preferred to employ a relatively low affinity, low capacity antibody or other binding protein. For the assessment of total Vitamin D, a high capacity, high affinity binder is preferred.

The invention, in another aspect, presents a product in the form of an immunoassay for the determination of 25-OH vitamin D in blood or blood components, wherein the assay makes use of a method according to any one of preceding embodiments. More particularly, such a product will be provided in the form of a kit for conducting the immunoassay. Such a kit may comprise the loose reagents involved, i.e. the binding protein and the labeled vitamin D compound. These reagents can be provided separately, and thus form a kit only upon their use in the assay of the invention. Preferably, the reagents are provided together, preferably packaged together, as one kit of parts. The kit optionally comprises a container for a sample of blood or blood components, but as is customary this may also be provided separately. Typically a kit comprises a binder immobilized on a solid phase and a separate conjugated vitamin D. Other kit components will depend, as is customary in the art, on the label chosen, as different labels may require different reagents.

The invention also pertains to a novel use of a binding protein, particularly an antibody for 25-OH vitamin D. This use in fact entails affecting the displacement of 25-OH vitamin D from vitamin D Binding Protein in an immunoassay for the determination of 25-OH vitamin D in blood or blood components. It will be understood that the displacement is not absolute, but is based on an equilibrium that sets in between binding of the vitamin D to endogenous vitamin D binding protein (DBP), and binding of vitamin D to the binding protein introduced in the assay.

It is to be understood that the invention is not limited to the embodiments and formulae as described hereinbefore. It is also to be understood that in the claims the word "comprising" does not exclude other elements or steps. Where an indefinite or definite article is used when referring to a singular noun e.g. "a" or "an", "the", this includes a plural of that noun unless something else is specifically stated.

The invention will be illustrated with reference to the following, non-limiting Example and the accompanying non-limiting Figure.

### Example

### Materials

Paramagnetic particles Magnetic particles (carboxyl, 4.7 µm) were coated with a polyclonal antibody against Vitamin-D. For this purpose, magnetic particles were resuspended in a 0.05M MES buffer of pH 6.2. To each mg of magnetic particle, at a concentration of 10 mg/ml, 2 µg of antibody and 3 µg of goat IgG was added. Subsequently 100 µg of 1-Ethyl-3-[3-dimethylaminopropyl]carbodiimide (EDC) was added and incubated on roller at ambient temperature (20-25 °C) for 1 hour. The particles were washed and resuspended in a 50 mM TRIS buffer of pH 8.0 containing 0.1M NaCl , preservatives, and sucrose.

The sample diluent consisted of 0.1M acetate buffer of pH 4.95 containing EDTA and preservatives.

The conjugate (i.e. the labeled vitamin D compound) is a biotinylated Vitamin-D that is complexed with an antibody against Biotin. Biotinylation of Vitamin-D in this example was through the C24 of the molecule. The antibody was labeled with acridinium. The conjugate was presented at a concentration of 0.5 mg/ml in a Bis-Tris buffer of pH 6.8 containing 0.5% Bovine gamma globulin, 0.9% NaCl, and 0.1% detergent and preservative.

### Protocol

The assay is performed as follows. In a cuvette, 20 µl of sample is pipetted together with 40 µl of sample diluent and 50 µl of magnetic particles. This mixture is incubated for 42 minutes at 37 °C. Subsequently, 50 µl of Acridinium Conjugate is added to the cuvette and incubated for 6 minutes. Again the magnetic particles are separated in a magnetic field and washed three times with wash buffer. A chemiluminescent signal was generated by addition of trigger reagent, for which purpose two separate solutions are added consisting of an alkaline peroxide solution and a chemiluminescence enhancer. The signal generated in the cuvette is inversely proportional to the concentration of 25 (OH)Vitamin D in the sample or calibrator. The concentration of 25(OH) vitamin D in the original sample can be calculated by comparing the signal of unknowns with the response of calibrators.

### Results

In the table below, a calibration curve is shown. 25(OH)Vitamin D3 calibrators that are Vitamin D-free serum based ranging from 0-160 ng/ml and serum pools (level 1-3) incubated with magnetic particles coated with anti-25(OH)vitamin D antibody in duplicate. After incubation the acridinium ester-labeled Vitamin D was added and after incubation and washing the particles, the signal was generated. The conjugated 25(OH) Vitamin D is displaced to a level of 23%. The signal of the levels 1-3 was read on the calibration curve using Graphpad software. The concentrations of the levels as derived from this calibrator curve is shown as "mean ng/ml" .

**Table**

| **Conc ng/ml** | 0 | 4 | 10 | 30 | 75 | 160 |
|---|---|---|---|---|---|---|
| RLU 1 | 60349 | 55921 | 49965 | 41575 | 25696 | 13409 |
| RLU 2 | 57648 | 55248 | 54699 | 42729 | 25603 | 13640 |
| Mean RLU | 58999 | 55585 | 52332 | 42152 | 25650 | 13525 |
| %CV | 3.2 | 0.9 | 6.4 | 1.9 | 0.3 | 1.2 |
| B/Bo% | | **94** | **89** | **71** | **43** | **23** |

| Levels | Target concentratio | Singl. 1 RLUs | Singl. 2 RLUs | Singl. 1 ng/ml | Singl. 2 ng/ml | Mean ng/ml | %CV |
|---|---|---|---|---|---|---|---|
| Level 1 | 20.0 | 43327 | 43220 | 26.9 | 27.1 | 27.0 | 0.5 |
| Level 2 | 40.0 | 38221 | 38349 | 37.6 | 37.3 | 37.4 | 0.5 |
| Level 3 | 75.0 | 31967 | 32870 | 53.6 | 51.0 | 52.3 | 3.5 |

## Claims

1. A method for assaying a sample of blood or blood components for the presence of 25-hydroxy vitamin D, comprising:
(a) adding a binding protein for 25-OH vitamin D to the sample;
(b) incubating the sample for an effective amount of time to allow a desired amount of 25-OH vitamin D to bind to the binding protein;
(c) subjecting the binding protein comprising 25-OH vitamin D bound thereto, to competitive binding with a labeled vitamin D compound;
(d) determining the concentration of labeled vitamin D compound bound to the binding protein.

2. A method according to claim 1, wherein the binding protein is an antibody.

3. A method according to claim 1, wherein the binding protein is vitamin D binding protein.

4. A method according to any one of the preceding claims, wherein the sample is diluted before incubation.

5. A method according to any one of the preceding claims, wherein the sample is human serum or plasma.

6. A method according to any one of the preceding claims, wherein the binding protein is provided in a form coated on magnetic particles.

7. A method according to any one of the preceding claims, wherein the incubation time is in a range of from 10 seconds to 10 minutes, preferably 30 seconds to 5 minutes.

8. A method according to any one of the preceding claims, wherein the labeled vitamin D compound comprises a label selected from the group consisting of radiolabels, fluorescent labels, luminescent labels, biotin labels, gold labels, enzyme labels.

9. A method according to any one of the preceding claims, wherein the concentration is determined with reference to a calibrator concentration for total 25-OH vitamin D.

10. A method according to any one of the claims 1-7, wherein the concentration is determined with reference to a calibrator concentration for bio-available 25-OH vitamin D.

11. An immunoassay for the determination of 25-OH vitamin D in blood or blood components, wherein the assay makes use of a method according to any one of the preceding claims.

12. A kit for conducting an immunoassay using the method of any one of the claims 1-9, the kit comprising a binding protein for 25-OH vitamin D immobilized on a solid phase, and a labeled vitamin D compound.

13. The use of a binding protein for 25-OH vitamin D, immobilized on a solid phase, for securing the displacement of 25-OH vitamin D from vitamin D Binding Protein in an immunoassay for the determination of 25-OH vitamin D in blood or blood components.

14. A use in accordance with claim 13, wherein the immunoassay is an immunoassay according to claim 11.
